# EUROPÄISCHE PATENTANMELDUNG

(11) **EP 2 684 936 A1**
(43) Veröffentlichungstag der Anmeldung: **15.01.2014**
(21) Anmeldenummer: 12176417.9
(22) Anmeldetag: 13.07.2012
(51) Int. Cl.: C10G 2/00, B01J 23/78, B01J 23/80, B01J 23/745, B01J 23/83

(54) **Verfahren zur Herstellung von Kohlenwasserstoffen aus Kohlendioxid und Wasserstoff und ein in dem Verfahren verwendbarer Katalysator**

(71) Anmelder: Wagner, Edmund, Dr.-Ing., 65187 Wiesbaden (DE)
(72) Erfinder: Wagner, Dr.-Ing. Edmund, 65187 Wiesbaden (DE); Baerns, Prof. em. Dr. Manfred, 14195 Berlin (DE); Rodemerck, Dr. Uwe, 18059 Rostock (DE); Smejkal, Dr. Quido, 12489 Berlin (DE); Barkschat, Dr. Axel, 18059 Rostock (DE); Holena, Dr. Martin, 18059 Rostock (DE); Linke, Dr. David, 18059 Rostock (DE); Marschall, Martina, 18059 Rostock (DE)
(74) Vertreter: HOFFMANN EITLE

(57) **Zusammenfassung**

Die Erfindung betrifft ein Verfahren zur Herstellung von Kohlenwasserstoffen aus Kohlendioxid und Wasserstoff, bei dem Kohlendioxid mit Wasserstoff in Gegenwart eines speziellen Katalysators umgesetzt wird. Mit dem erfindungsgemäßen Verfahren können als Treibstoffe (Benzin, Diesel, Kerosin) interessante C₅-C₁₅ Kohlenwasserstoffe mit sehr hoher Selektivität gebildet werden. Ferner entstehen in dem Verfahren mit guter Selektivität C₂-C₄ Kohlenwasserstoffe, die als wertvolle Ausgangsstoffe in der chemischen Industrie verwendet werden können. In dem Verfahren wird ein hoher Umsatz von CO₂ bereits ohne Rückführung von überschüssigem Kohlendioxid erreicht. Gemäß einem weiteren Aspekt betrifft die Erfindung die Katalysatoren als solche.

## Beschreibung

### Gebiet der Erfindung

Die vorliegende Erfindung betrifft ein Verfahren zur Herstellung von Kohlenwasserstoffen aus Kohlendioxid und Wasserstoff, bei dem Kohlendioxid mit Wasserstoff in Gegenwart eines speziellen Katalysators umgesetzt wird. Mit dem erfindungsgemäßen Verfahren können als Treibstoffe (Benzin, Diesel, Kerosin) interessante C₅-C₁₅ Kohlenwasserstoffe mit sehr hoher Selektivität gebildet werden. Ferner entstehen in dem Verfahren mit guter Selektivität C₂-C₄ Kohlenwasserstoffe, die als wertvolle Ausgangsstoffe in der chemischen Industrie verwendet werden können. Bei dem Verfahren wird ein hoher Umsatz von CO₂ bereits ohne Rückführung von überschüssigem Kohlendioxid erreicht. Gemäß einem weiteren Aspekt betrifft die Erfindung die Katalysatoren als solche.

### Stand der Technik

Die weltweit zurückgehenden Vorräte an fossilen Kohlenstoffträgern sowie die zunehmende Anreicherung der Atmosphäre mit Kohlendioxid (CO₂) und der dadurch bedingte Treibhauseffekt (globale Erwärmung) erfordern ein Umdenken bei der Nutzung von fossilem Kohlenstoff als Energieträger und Ausgangsstoff für die Produktion von Treibstoffen. Gegenwärtig erfolgt ein Umstieg der Treibstoffproduktion von Erdöl- zu Erdgas-basierten Verfahren. Dazu werden die Synthesegaserzeugung aus Methan und die katalysierte Fischer-Tropsch-Synthese (FT) gekoppelt.

Fischer-Tropsch-Synthese:

CO + 2 H₂ → [-CH₂-] + H₂O (1)

Als Produkte können kürzerkettige (Benzin, Diesel) und längerkettige Kohlenwasserstoffe (Wachse) erhalten werden. Benzin enthält Kohlenwasserstoffe mit 5 bis 12 Kohlenstoffatomen, während Diesel längerkettige Kohlenwasserstoffe enthält, nämlich ab ca. 9 Kohlenstoffatomen.

Die FT-Synthese trägt selbst zur CO₂-Emission bei, da CO₂ als Nebenprodukt in mehreren Gleichgewichtsreaktionen entsteht, z.B. aus Kohlenmonoxid und Wasserdampf im Wassergas-Shift:

CO + H₂O ⇆ CO₂ + H₂ (2)

Deshalb wird nach Verfahren zur Treibstoffproduktion gesucht, die CO₂-neutral sind. Beispielsweise eignet sich hier CO₂ als Kohlenstoffquelle, da CO₂ bei der Produktion verbraucht wird und beim Verbrennen des gewonnenen Treibstoffs lediglich die vorher gebundene Menge CO₂ freigesetzt wird.

Ein Ansatz hierzu ist, CO₂ mit Wasserstoff im umgekehrten Wassergas-Shift (reverse water-gas shift = RWGS) zu CO und Wasser umzusetzen:

CO₂ + H₂ ⇆ CO + H₂O (3)

und anschließend das gewonnene CO in der Fischer-Tropsch-Reaktion durch Hydrierung in Kohlenwasserstoffe umzuwandeln. Die Gesamtreaktion lautet dann:

CO₂ + 3 H₂ → [-CH₂-] + 2 H₂O (4)

Die RWGS bzw. deren umgekehrte Reaktion ist eine Gleichgewichtsreaktion, die durch die Temperatur und das Verhältnis der Partialdrücke limitiert ist. Ein H₂:CO₂-Verhältnis von 3 entspricht dem stöchiometrischen Verhältnis der Gesamtreaktion, siehe Gleichung (4). Der CO₂-Umsatz und die Ausbeute von CO steigen mit der Temperatur; erst bei ca. 500°C werden 50% des CO₂ zu CO umgewandelt und für einen vollständigen Umsatz werden Temperaturen von weit über 1000°C benötigt. Im Temperaturbereich von 220-300°C liegt der Anteil des zu CO umgewandelten CO₂ bei 12-22%.

Neben der Treibstoffproduktion ist der größte Verbraucher von fossilen Kohlenstoffträgern in der chemischen Industrie die Polymerproduktion. Hier werden vorwiegend kurzkettige Olefine (Ethylen, Propylen, Butene) als Ausgangsstoffe eingesetzt, aus denen direkt oder nach Modifizierung Polymere, wie Polyethylen, Polypropylen, Polyacrylate und Polyurethane, hergestellt werden. Gegenwärtig werden die genannten Olefine hauptsächlich aus fossilen Rohstoffen in Raffinerien durch Dampfcracken hergestellt. Andere Verfahren, wie die katalytische Dehydrierung von Alkanen oder die Umwandlung von Olefinen in andere Olefine durch Metathese spielen nur eine untergeordnete Rolle und basieren ebenfalls auf fossilen Rohstoffen. Um von fossilen Rohstoffen unabhängig zu werden, ist es erstrebenswert, Kohlenwasserstoffe für die Treibstoffproduktion und kurzkettige Olefine aus dem Rohstoff CO₂ herzustellen.

Ein weiterer Ansatz, die Anreicherung von CO₂ in der Atmosphäre und den dadurch bedingten Treibhauseffekt (globale Erwärmung) zu reduzieren, ist CO₂ in unterirdischen Kavernen zu speichern. Dabei könnte allerdings das CO₂ mit dem Gestein reagieren und die Stabilität der Kavernen gefährden.

Es wäre wünschenswert, das CO₂ in flüssige Kohlenwasserstoffe (mehr als 5 Kohlenstoffatome) umzuwandeln, die dann in den Kavernen gespeichert werden und bei Bedarf als Treibstoff- oder Rohstoffresource für die chemische Industrie verwendet werden können.

In einer frühen Arbeit von G. D. Weatherbee et al. (J. Catal. 87 (1984) 352) wurde die Hydrierung von CO₂ durch Cobalt, Eisen und Ruthenium beschrieben, wobei diese auf Silica geträgert waren. In D. Li et al., Appl. Catal. A 180 (1999) 227 wurde die Methanerzeugung aus CO₂ und H₂ in Gegenwart von Ruthenium auf einem TiO₂-Träger untersucht.

Verschiedene Publikationen von T. Riedel et al. und H. Schulz et al. (T. Riedel, Dissertation Universität Karlsruhe 2002; H. Schulz et al., Appl. Catal. A, 186 (1999) 215; T. Riedel et al., Appl. Catal. A, 186 (1999) 201; T. Riedel et al., Ind. Eng. Chem. Res., 40 (2001) 1355; H. Schulz et al., Topics Catal., 32 (2005) 117) befassen sich mit der Hydrierung von CO₂ durch Fe/Al₂O₃-Katalysatoren, die die Promotoren Kupfer und Kalium enthalten. Des Weiteren sind in T. Riedel et al., Appl. Catal. A, 186 (1999) 201 neben den Eisenkatalysatoren auf Al₂O₃-Trägern auch solche auf TiO₂-oder SiO₂-Trägern für die Hydrierung von CO₂ beschrieben. In G. Kishan et al., Catal. Lett. 56 (1998) 215 wurde Fe-K auf Al₂O₃, MgO oder Al₂O₃-MgO, mit verschiedenen Anteilen an Al₂O₃ und MgO, in der Hydrierung von CO₂ verwendet. Eisenkatalysatoren, die Cer als Promotor enthalten können, wurden in S.-S. Nam et al., J. Chem. Res. (1999) 344 und S.-S. Nam et al., Appl. Catal. A 179 (1999) 155 auf Alkalimetallion-getauschte Zeolithe aufgebracht und in der Hydrierung von CO₂ getestet. Eine Veröffentlichung von R.W. Dorner et al. (R.W. Dorner et al., "Effects of Loading and Doping on Iron-based CO2 Hydrogenation Catalysts", Naval Research Laboratory, NRL/MR/6180--09-9200 (2009)) befasst sich mit der CO₂-Hydrierung in Gegenwart von auf γ-Al₂O₃-geträgerten Eisenkatalysatoren, die mit Mangan dotiert sein können. Der Katalysator 100Fe6, 6Cu15, 7Al4K wurde in S.-R. Yan et al., Appl. Catal. A 194-195 (2000) 63-70 bezüglich seiner Aktivität in der Hydrierung von CO₂ getestet.

Die nicht sehr umfangreiche Literatur auf dem Gebiet der katalytischen CO₂-Hydrierung ist in P.S.S. Prasad et al., Catal. Surv. Asia 12 (2008) 170 zusammengefasst.

Das Ziel der Erfindung ist es, ein Verfahren zur Herstellung von Kohlenwasserstoffen und einen Katalysator dafür bereitzustellen, mit dem eine hohe Selektivität für Kohlenwasserstoffe, insbesondere der Kettenlängen C₅-C₁₅ aber auch der Kettenlängen C₂-C₄ bei einem möglichst hohen Umsatz von CO₂ bereits ohne Rückführung von überschüssigem CO₂ erzielt wird. Insbesondere wird nach einem Verfahren gesucht, mit dem eine höhere Selektivität für flüssige C₅-C₁₅ Kohlenwasserstoffe, die beispielsweise als Treibstoffe geeignet sind, und ein höherer CO₂-Umsatz als mit den Katalysatoren des Standes der Technik, beispielsweise dem 100Fe6, 6Cu15, 7Al4K von S.-R. Yan et al. in Appl. Catal. A 194-195 (2000) 63-70, erreicht werden.

### Zusammenfassung der Erfindung

Die Aufgabe wird erfindungsgemäß durch ein Verfahren zur Herstellung von Kohlenwasserstoffen aus Kohlendioxid und Wasserstoff in Gegenwart eines speziellen Katalysators gelöst, wie es in dem beigefügten Patentanspruch 1 definiert ist. Der in dem erfindungsgemäßen Verfahren verwendete Katalysator enthält Eisen und mindestens ein Alkalimetall und erfüllt ferner mindestens eines der folgenden Merkmale (i) bis (iii) :
(i) der Katalysator enthält, bezogen auf das Gesamtgewicht des Katalysators, Eisen in einer Menge von mindestens 10 Gew.-%, vorzugsweise von 15 und 35 Gew.-%, und ferner Kupfer und mindestens einen weiteren Vertreter, ausgewählt aus Magnesium, Zink, Lanthan und Zirkonium;
(ii) der Katalysator umfasst TiO₂ und/oder ein Aluminium-Magnesiumhydroxycarbonat als Katalysatorträger;
(iii) der Katalysator enthält ferner Ruthenium und/oder Cobalt.

Eine spezielle Ausführungsform des obigen Katalysators mit dem Merkmal (i) ist Gegenstand des beigefügten Patentanspruchs 13.

Vorteilhafte Weiterbildungen des erfindungsgemäßen Verfahrens zur Herstellung von Kohlenwasserstoffen aus Kohlendioxid und Wasserstoff und des erfindungsgemäßen Katalysators sind Gegenstand der Unteransprüche.

### Ausführliche Beschreibung der Erfindung

In dem erfindungsgemäßen Katalysator und im Katalysator des erfindungsgemäßen Verfahrens können die Metalle, wie Eisen, Alkalimetall (z.B. Lithium, Natrium und Kalium), Kupfer, Magnesium, Zink, Lanthan, Aluminium, Zirkonium, Mangan, Ruthenium und Cobalt auch als Verbindungen, insbesondere als Oxide vorliegen. Vorzugsweise liegt zumindest ein Teil der Metalle, besonders bevorzugt liegen sämtliche Metalle als Oxid vor.

Im erfindungsgemäßen Verfahren zur Herstellung von Kohlenwasserstoffen aus Kohlendioxid und Wasserstoff, bei dem Kohlendioxid mit Wasserstoff in Gegenwart eines Katalysators umgesetzt wird, wobei der Katalysator Eisen und mindestens ein Alkalimetall enthält.

Das mindestens eine Alkalimetall ist vorteilhaft ausgewählt aus der Gruppe bestehend aus Lithium, Natrium, Kalium und Rubidium. Vorzugsweise enthält der Katalysator Kalium als das mindestens eine Alkalimetall. Vorzugsweise ist der Anteil an dem mindestens einen Alkalimetall 1 - 30 Gew.-% und besonders bevorzugt 5 - 12 Gew.-% in Bezug auf den Eisenanteil im Katalysator. Wenn der Katalysator im erfindungsgemäßen Verfahren Kalium enthält, ist der Kaliumgehalt vorzugsweise 1 - 30 Gew.-% und besonders bevorzugt 5 - 12 Gew.-% in Bezug auf den Eisenanteil im Katalysator.

Im erfindungsgemäßen Verfahren ist wesentlich, dass der Katalysator mindestens eines der folgenden Merkmale (i) bis (iii) erfüllt:
(i) der Katalysator enthält, bezogen auf das Gesamtgewicht des Katalysators, Eisen in einer Menge von mindestens 10 Gew.-%, vorzugsweise von 15 und 35 Gew.-%, und ferner Kupfer und mindestens einen weiteren Vertreter, ausgewählt aus Magnesium, Zink, Lanthan und Zirkonium;
(ii) der Katalysator umfasst TiO₂ und/oder ein Aluminium-Magnesiumhydroxycarbonat als Katalysatorträger;
(iii) der Katalysator enthält ferner Ruthenium und/oder Cobalt.

Eine Ausführungsform des erfindungsgemäßen Verfahrens betrifft die Verwendung eines Katalysators, der das Merkmal (i) erfüllt (Verfahren (i)).

Vorzugsweise ist das mindestens eine Alkalimetall, das im Katalysator des Verfahrens (i) enthalten ist, Kalium. Optional kann der Katalysator des Verfahrens (i) zwei weitere Vertreter, ausgewählt aus Magnesium, Zink, Lanthan und Zirkonium, enthalten. Der Katalysator des Verfahrens (i) kann außerdem Mangan und/oder Aluminium enthalten. Beispielsweise kann der Katalysator des Verfahrens (i) Mangan und Kupfer enthalten. In bevorzugten Ausführungsformen des erfindungsgemäßen Verfahrens enthält der Katalysator:
- Kalium und Lanthan;
- Kalium und Magnesium;
- Kalium und Zirkonium;
- Kalium und Zink;
- Kalium, Magnesium und Mangan; oder
- Kalium, Zirkonium und Mangan,
und besonders bevorzugt sind dabei:
- Kalium, Magnesium und Mangan; oder
- Kalium, Zirkonium und Mangan.

Die Komponenten Kalium, Kupfer, Magnesium, Zink, Lanthan, Zirkonium, Mangan und Aluminium stellen dabei Promotoren dar. Für die Zwecke der vorliegenden Anmeldung ist ein Promotor als Bestandteil bzw. Komponente eines Katalysators definiert, der die Katalysereaktion positiv beeinflusst.

Das Gewichtsverhältnis von Eisen zum Gesamtgewicht aller Promotoren im Katalysator des Verfahrens (i) ist vorzugsweise zwischen 1,5 und 15.

Vorzugsweise enthält der Katalysator des Verfahrens (i) zusätzlich Ruthenium. Die Komponenten Eisen und Ruthenium stellen Aktivkomponenten dar. Für die Zwecke der vorliegenden Anmeldung ist eine Aktivkomponente als der katalytisch aktive Bestandteil bzw. Komponente definiert.

Der Katalysator des Verfahrens (i) kann einen Katalysatorträger umfassen, auf dem die Aktivkomponenten und Promotoren aufgebracht sind (geträgerter Katalysator).

Falls der Katalysator des Verfahrens (i) keinen Katalysatorträger umfasst (ungeträgerter Katalysator), ist das Gesamtgewicht aller Promotoren im Katalysator bezogen auf den Katalysator insgesamt vorzugsweise zwischen 15 und 50 Gew.-% und besonders bevorzugt zwischen 30 und 40 Gew.-%. Besonders bevorzugte Beispiele des ungeträgerten Katalysators des Verfahrens (i) sind:
11, 3La14, 1Cu70, 7Fe3, 9K; und
11, 6Mg11, 6Mn11, 6Cu58, 2Fe7, 0K,
wobei die Metalle als Oxid vorliegen können und die Anteile der Metalle in Gew.-%, bezogen auf das Gesamtgewicht aller Metalle in metallischer Form im Katalysator angegeben sind. Vorzugsweise liegt zumindest ein Teil der Metalle, besonders bevorzugt liegen sämtliche Metalle als Oxid vor. Ist der Katalysator des Verfahrens (i) geträgert, so kann der Katalysatorträger aus der Gruppe bestehend aus Al₂O₃, Al₂O₃-MgO, TiO₂, La₂O₃, ZrO₂, ZrO₂-La₂O₃, ZrO₂-SiO₂, Zeolithen und Aluminium-Magnesiumhydroxycarbonaten ausgewählt sein. Al₂O₃-MgO bezeichnet Mischungen aus Al₂O₃ und MgO, wobei das Mischungsverhältnis beliebig sein kann. ZrO₂-La₂O₃ bezeichnet Mischungen aus ZrO₂ und La₂O₃, wobei das Mischungsverhältnis beliebig sein kann, bevorzugt liegt das Gewichtsverhältnis von La₂O₃: ZrO₂ zwischen 5: 95 und 15: 85. ZrO₂-SiO₂ bezeichnet Mischungen aus ZrO₂ und SiO₂, wobei das Mischungsverhältnis beliebig sein kann. In den Aluminium-Magnesiumhydroxycarbonaten kann das Gewichtsverhältnis zwischen Al₂O₃ und MgO beliebig sein. Bevorzugt liegt das Gewichtsverhältnis von Al₂O₃: MgO zwischen 30: 70 (z.B. Pural MG70) und 50: 50. Die Aluminium-Magnesiumhydroxycarbonate umfassen amorphe und kristalline Formen, unter den kristallinen Formen ist Hydrotalcit bevorzugt.

Bevorzugte Katalysatorträger sind TiO₂, Al₂O₃ und Aluminium-Magnesiumhydroxycarbonat, wobei TiO₂ besonders bevorzugt ist. Die Trägermaterialien Al₂O₃ und TiO₂ sind hinsichtlich der Ausbeute an C₅-C₁₅ Kohlenwasserstoffen vorteilhaft. Wird TiO₂ als Katalysatorträger im Katalysator des Verfahrens (i) verwendet, so wird eine hohe Ausbeute an C₅-C₁₅ Kohlenwasserstoffen erzielt, während gleichzeitig wenig Methan gebildet wird. Der Katalysatorträger hat vorzugsweise eine Partikelgröße von 100 bis 250 µm.

Das Gesamtgewicht aller Promotoren im Katalysator des Verfahrens (i) beträgt vorzugsweise zwischen 1,5 und 20 Gew.-% und besonders bevorzugt zwischen 3 und 10 Gew.-% bezogen auf das Gewicht des Katalysators insgesamt.

Der geträgerte Katalysator des Verfahrens (i) ist vorzugsweise aus den folgenden Katalysatoren ausgewählt:
0,07Ru 1,27Mg 1,78Cu 20Fe 1,74K / Al₂O₃;
0,1Ru 0,26Mg 20Fe 2,1K / Al₂O₃;
0,08Ru 0,95Mg 2,49Cu 20Fe 1,52K / Al₂O₃;
0,17Ru 1,15Mg 2,76Cu 20Fe 1,82K / Al₂O₃;
0,17Ru 1,15Mg 2,19Cu 20Fe 1,52K / Al₂O₃;
0,02Ru 1,02Mg 0,1Zr 20Fe 0,83K / Al₂O₃;
0,63La 4Cu 20Fe 1,31K / Al₂O₃;
0,07Ru 0,3Zn 20Fe 2,1K / TiO₂;
2,23Zr 4Cu 20Fe 1,73K / TiO₂;
0,1Ru 0,2Cu 0,29Zn 20Fe 1,4K / TiO₂;
1,01 Cu 20Fe 1,7K / TiO₂;
2,3Zr 1,8Mn 2,6Zn 20Fe 1,7K / TiO₂;
3,27Zr 2,96Mn 1Cu 20Fe 1,48K / TiO₂;
20Fe 2,66A1 2,14Cu 1,78K / TiO₂;
3,18La 4Cu 20Fe 1,1K / TiO₂;
20Fe 1,78Cu 1,74K 1,27Mg 0,07Ru / Aluminium-Magnesiumhydroxycarbonat;
20Fe 4Mg 4Mn 4Cu 2,4K / Aluminium-Magnesiumhydroxycarbonat; und
20Fe 2,1K 0,3Zn 0,07Ru / Aluminium-Magnesiumhydroxycarbonat, wobei die Metalle als Oxid vorliegen können und die Anteile der Metalle in Gew.-%, bezogen auf die Summe des Gesamtgewichts aller Metalle in metallischer Form im Katalysator und des Katalysatorträgers angegeben sind. Vorzugsweise liegt zumindest ein Teil der Metalle, besonders bevorzugt liegen sämtliche Metalle als Oxid vor.

Eine andere Ausführungsform des erfindungsgemäßen Verfahrens betrifft die Verwendung eines Katalysators, der das Merkmal (ii) erfüllt (Verfahren (ii)). Wird im Katalysator des erfindungsgemäßen Verfahrens TiO₂ oder Aluminium-Magnesiumhydroxycarbonate als Katalysatorträger verwendet, so wird eine besonders hohe Ausbeute an wertvollen Kohlenwasserstoffen, wie beispielsweise C₂-C₄ und C₅-C₁₅ Kohlenwasserstoffen, erzielt, während gleichzeitig wenig unerwünschtes Methan gebildet wird. Die Katalysatorträger TiO₂ und Aluminium-Magnesiumhydroxycarbonate haben vorzugsweise eine Partikelgröße von 100 bis 250 µm.

Der Anteil an Eisen im Katalysator, bezogen auf den Katalysator insgesamt, ist vorzugsweise zwischen 10 und 50 Gew.-% Eisen, besonders bevorzugt zwischen 15 und 35 Gew.-% Eisen und ganz besonders bevorzugt zwischen 20 und 25 Gew.-% Eisen. Durch diesen hohen Eisenanteil wird ein besonders hoher CO₂-Umsatz erreicht.

Vorzugsweise enthält der Katalysator im Verfahren (ii) Kalium als das mindestens eine Alkalimetall. In einer bevorzugten Ausführungsform des Verfahrens (ii) enthält der Katalysator mindestens einen weiteren Vertreter, wobei dieser aus der Gruppe bestehend aus Magnesium, Zink, Lanthan, Aluminium, Zirkonium, Mangan und Kupfer ausgewählt ist. Optional können zwei, drei oder vier weitere Vertreter im Katalysator des Verfahrens (ii) enthalten sein. Bevorzugt ist der mindestens eine weitere Vertreter Magnesium, Zink oder Kupfer. Besonders bevorzugt enthält der Katalysator im Verfahren (ii) Kalium und Kupfer und mindestens einen weiteren Vertreter, der ausgewählt ist aus der Gruppe bestehend aus Aluminium, Lanthan, Zirkonium, Zink und Magnesium. In bevorzugten Ausführungsformen des Verfahrens (ii) enthält der Katalysator:
- Kalium und Zink;
- Kalium und Magnesium;
- Kalium und Kupfer;
- Kalium, Kupfer und Zirkonium;
- Kalium, Kupfer und Zink;
- Kalium, Kupfer und Aluminium;
- Kalium, Kupfer und Lanthan;
- Kalium, Kupfer, Magnesium und Mangan;
- Kalium, Zirkonium, Mangan und Zink; oder
- Kalium, Kupfer, Zirkonium und Mangan,

Und besonders bevorzugt sind dabei:
- Kalium, Zirkonium, Mangan und Zink; oder
- Kalium, Kupfer, Zirkonium und Mangan.

Die Komponenten Kalium, Kupfer, Magnesium, Zink, Lanthan, Zirkonium, Mangan und Aluminium stellen dabei Promotoren dar.

Das Gewichtsverhältnis von Eisen zum Gesamtgewicht aller Promotoren im Katalysator des Verfahrens (ii) ist vorzugsweise zwischen 1,5 und 15.

Das Gesamtgewicht aller Promotoren im Katalysator des Verfahrens (ii) beträgt vorzugsweise zwischen 1,5 und 20 Gew.-% und besonders bevorzugt zwischen 3 und 10 Gew.-% bezogen auf das Gewicht des Katalysators insgesamt.

Vorzugsweise enthält der Katalysator des Verfahrens (ii) zusätzlich Ruthenium. Die Komponenten Eisen und Ruthenium stellen Aktivkomponenten dar.

Beispiele der Katalysatoren des Verfahrens (ii) sind:
0,07Ru 0,3Zn 20Fe 2,1K / TiO₂;
0,2Ru 20Fe 1,99K / TiO₂;
0,2Ru 20Fe 1,52K / TiO₂;
2,23Zr 4Cu 20Fe 1,73K / TiO₂;
0,1Ru 0,2Cu 0,29Zn 20Fe 1,4K / TiO₂;
1,01Cu 20Fe 1,7K / TiO₂;
2,3Zr 1,8Mn 2,6Zn 20Fe 1,7K / TiO₂;
0,2Ru 20Fe 0,98K / TiO₂;
3,27Zr 2,96Mn 1Cu 20Fe 1,48K / TiO₂;
0,2Ru 20Fe 1,16K / TiO₂;
20Fe 2,66Al 2,14Cu 1,78K / TiO₂; und
3,18La 4Cu 20Fe 1,1K / TiO₂,
wobei die Metalle als Oxid vorliegen können und die Anteile der Metalle in Gew.-%, bezogen auf die Summe des Gesamtgewichts aller Metalle in metallischer Form im Katalysator und des Katalysatorträgers angegeben sind. Vorzugsweise liegt zumindest ein Teil der Metalle, besonders bevorzugt liegen sämtliche Metalle als Oxid vor.

Eine weitere Ausführungsform des erfindungsgemäßen Verfahrens betrifft die Verwendung eines Katalysators, der das Merkmal (iii) erfüllt (Verfahren (iii)). In dieser Ausführungsform stellen die Komponenten Eisen, Ruthenium und Cobalt Aktivkomponenten dar.

Vorzugsweise enthält der Katalysator des Verfahrens (iii) Kalium als das mindestens eine Alkalimetall. In einer bevorzugten Ausführungsform des Verfahrens (iii) enthält der Katalysator mindestens einen weiteren Vertreter, wobei dieser aus der Gruppe bestehend aus Magnesium, Zink, Lanthan, Aluminium, Zirkonium, Mangan und Kupfer ausgewählt ist. Optional können zwei, drei oder vier weitere Vertreter im Katalysator des Verfahrens (iii) enthalten sein. Bevorzugt ist der mindestens eine weitere Vertreter Magnesium, Zink oder Kupfer. Besonders bevorzugt enthält der Katalysator im Verfahren (iii) Kalium und Kupfer und mindestens einen weiteren Vertreter, der ausgewählt ist aus der Gruppe bestehend aus Aluminium, Lanthan, Zirkonium, Zink und Magnesium. In besonders bevorzugten Ausführungsformen des Verfahrens (iii) enthält der Katalysator:
- Kalium und Zink;
- Kalium und Magnesium;
- Kalium und Kupfer;
- Kalium, Zirkonium, Mangan und Zink;
- Kalium, Kupfer, Magnesium und Mangan; oder
- Kalium, Kupfer, Zirkonium und Mangan.

Die Komponenten Kalium, Kupfer, Magnesium, Zink, Lanthan, Zirkonium, Mangan und Aluminium stellen dabei Promotoren dar. Das Gewichtsverhältnis von Eisen zum Gesamtgewicht aller Promotoren im Katalysator des Verfahrens (iii) ist vorzugsweise zwischen 1,5 und 15.

Der Katalysator im Verfahren (iii) kann sowohl geträgert als auch ungeträgert sein.

Falls der Katalysator des Verfahrens (iii) ein ungeträgerter ist, ist das Gesamtgewicht aller Promotoren im Katalysator bezogen auf den Katalysator insgesamt vorzugsweise zwischen 15 und 50 Gew.-% und besonders bevorzugt zwischen 30 und 40 Gew.-%.

Wenn der Katalysator des Verfahrens (iii) geträgert ist - dies ist eine bevorzugte Ausführungsform -, dann kann der Katalysatorträger aus der Gruppe bestehend aus Al₂O₃, Al₂O₃-MgO, TiO₂, La₂O₃, ZrO₂, ZrO₂-La₂O₃, ZrO₂-SiO₂, Zeolithen und Aluminium-Magnesiumhydroxycarbonaten ausgewählt sein. Al₂O₃-MgO bezeichnet Mischungen aus Al₂O₃ und MgO, wobei das Mischungsverhältnis beliebig sein kann. ZrO₂-La₂O₃ bezeichnet Mischungen aus ZrO₂ und La₂O₃, wobei das Mischungsverhältnis beliebig sein kann, bevorzugt liegt das Gewichtsverhältnis von La₂O₃ : ZrO₂ zwischen 5: 95 und 15: 85. ZrO₂-SiO₂ bezeichnet Mischungen aus ZrO₂ und SiO₂, wobei das Mischungsverhältnis beliebig sein kann. In den Aluminium-Magnesiumhydroxycarbonaten kann das Gewichtverhältnis zwischen Al₂O₃ und MgO beliebig sein. Bevorzugt liegt das Gewichtsverhältnis von Al₂O₃. MgO zwischen 30 : 70 (z.B. Pural MG70) und 50 : 50. Die Aluminium-Magnesiumhydroxycarbonate umfassen amorphe und kristalline Formen, unter den kristallinen Formen ist Hydrotalcit bevorzugt.

Bevorzugte Katalysatorträger sind TiO₂, Al₂O₃ und Aluminium-Magnesiumhydroxycarbonat, wobei TiO₂ besonders bevorzugt ist. Diese Trägermaterialien sind hinsichtlich der Ausbeute an C₅-C₁₅ Kohlenwasserstoffen vorteilhaft. Der Katalysatorträger hat vorzugsweise eine Partikelgröße von 100 bis 250 µm.

Das Gesamtgewicht aller Promotoren im Katalysator des Verfahrens (iii) beträgt vorzugsweise zwischen 1,5 und 20 Gew.-% und besonders bevorzugt zwischen 3 und 10 Gew.-% bezogen auf das Gewicht des Katalysators insgesamt.

Im Verfahren (iii) ist der folgende Katalysator besonders bevorzugt:
0,2Ru 18,08Fe 2,17K 1,92Co / Al₂O₃,
wobei die Metalle als Oxid vorliegen können und die Anteile der Metalle in Gew.-%, bezogen auf die Summe des Gesamtgewichts aller Metalle in metallischer Form im Katalysator und des Katalysatorträgers angegeben sind. Vorzugsweise liegt zumindest ein Teil der Metalle, besonders bevorzugt liegen sämtliche Metalle als Oxid vor.

Eine weitere Ausführungsform des erfindungsgemäßen Verfahrens (Verfahren (iv)) betrifft die Herstellung von Kohlenwasserstoffen aus Kohlendioxid und Wasserstoff, bei dem Kohlendioxid mit Wasserstoff in Gegenwart eines Katalysators umgesetzt wird, wobei der Katalysator Eisen, Kalium, Kupfer und Magnesium enthält, wobei Eisen in einer Menge von mindestens 8 Gew.-%, vorzugsweise zwischen 10 und 50 Gew.-%, besonders bevorzugt zwischen 15 und 35 Gew.-%, bezogen auf das Gesamtgewicht des Katalysators, enthalten ist. Der Katalysator kann außerdem Mangan enthalten. Vorzugsweise ist der Anteil an Kalium 1 - 30 Gew.-% und besonders bevorzugt 5 - 12 Gew.-% in Bezug auf den Eisenanteil im Katalysator. Die Komponenten Kalium, Kupfer, Magnesium, und Mangan stellen dabei Promotoren dar. Eisen ist als Aktivkomponente enthalten.

Das Gewichtsverhältnis von Eisen zum Gesamtgewicht aller Promotoren im Katalysator des Verfahrens ist vorzugsweise zwischen 1,5 und 15.

Der Katalysator des Verfahrens kann einen Katalysatorträger umfassen, auf dem die Aktivkomponenten und Promotoren aufgebracht sind (geträgerter Katalysator).

Falls der Katalysator des Verfahrens keinen Katalysatorträger umfasst (ungeträgerter Katalysator), ist das Gesamtgewicht aller Promotoren im Katalysator bezogen auf den Katalysator insgesamt vorzugsweise zwischen 15 und 50 Gew.-% und besonders bevorzugt zwischen 30 und 40 Gew.-%.

Ist der Katalysator des Verfahrens geträgert, so kann der Katalysatorträger aus der Gruppe bestehend aus Al₂O₃, Al₂O₃-MgO, TiO₂, La₂O₃, ZrO₂, ZrO₂-La₂O₃, ZrO₂-SiO₂, Zeolithen und Aluminium-Magnesiumhydroxycarbonaten ausgewählt sein. Al₂O₃-MgO bezeichnet Mischungen aus Al₂O₃ und MgO, wobei das Mischungsverhältnis beliebig sein kann. ZrO₂-La₂O₃ bezeichnet Mischungen aus ZrO₂ und La₂O₃, wobei das Mischungsverhältnis beliebig sein kann, bevorzugt liegt das Gewichtsverhältnis von La₂O₃ : ZrO₂ zwischen 5: 95 und 15: 85. ZrO₂-SiO₂ bezeichnet Mischungen aus ZrO₂ und SiO₂, wobei das Mischungsverhältnis beliebig sein kann. In den Aluminium-Magnesiumhydroxycarbonaten kann das Gewichtverhältnis zwischen Al₂O₃ und MgO beliebig sein. Bevorzugt liegt das Gewichtsverhältnis von Al₂O_{3 :} MgO zwischen 30 : 70 (z.B. Pural MG70) und 50: 50. Die Aluminium-Magnesiumhydroxycarbonate umfassen amorphe und kristalline Formen, unter den kristallinen Formen ist Hydrotalcit bevorzugt.

Der Katalysatorträger ist vorzugsweise Al₂O₃. Der Katalysatorträger hat vorzugsweise eine Partikelgröße von 100 bis 250 µm.

Das Gesamtgewicht aller Promotoren im Katalysator des Verfahrens beträgt vorzugsweise zwischen 1,5 und 20 Gew.-% und besonders bevorzugt zwischen 3 und 10 Gew.-% bezogen auf das Gewicht des Katalysators insgesamt.

Die folgenden Katalysatoren sind besonders bevorzugt:
10Fe 1,0Mg 2,0Cu 0,8K / Al₂O₃
10Fe 2,0Mg 2,0Mn 2,0Cu 1,2K / Al₂O₃
wobei die Metalle als Oxid vorliegen können und die Anteile der Metalle in Gew.-%, bezogen auf die Summe des Gesamtgewichts aller Metalle in metallischer Form im Katalysator und des Katalysatorträgers angegeben sind. Vorzugsweise liegt zumindest ein Teil der Metalle, besonders bevorzugt liegen sämtliche Metalle als Oxid vor.

Ein weiterer Aspekt der Erfindung betrifft die Verwendung der Katalysatoren, wie oben beschrieben, in dem erfindungsgemäßen Verfahren, wie es beispielsweise in dem beigefügten Patentanspruch 1 definiert ist.

Im Folgenden wird das erfindungsgemäße Verfahren allgemein beschrieben. Das erfindungsgemäße Verfahren betrifft die Umsetzung von Kohlendioxid mit Wasserstoff in Gegenwart eines Katalysators, d.h. die Hydrierung von Kohlendioxid durch Wasserstoff in Gegenwart eines Katalysators zu Kohlenwasserstoffen. Dabei findet vorzugsweise die RWGS-Reaktion von Kohlendioxid zu Kohlenmonoxid statt und das gebildete Kohlenmonoxid wird in einer Fischer-Tropsch-Synthese zu Kohlenwasserstoffen umgesetzt. Somit kann das erfindungsgemäße Verfahren auch allgemein als Fischer-Tropsch-Verfahren bezeichnet werden. Bevorzugt sind die Kohlenwasserstoffe C₅-C₁₅ Kohlenwasserstoffe und/oder C₂-C₄ Kohlenwasserstoffe.

Die Umsetzung des Kohlendioxids mit Wasserstoff im erfindungsgemäßen Verfahren findet vorzugsweise bei einer Temperatur von 150 bis 400°C, besonders bevorzugt bei 300 bis 370°C, noch mehr bevorzugt bei 320°C bis 370°C und ganz besonders bevorzugt bei 350°C statt. Eine Temperatur von 150 bis 400°C führt zu einer besonders hohen Ausbeute an Kohlenwasserstoffen, insbesondere C₅-C₁₅ Kohlenwasserstoffen. Eine Temperatur von 300°C bis 350°C ist in Bezug auf die Ausbeute an C₅-C₁₅ Kohlenwasserstoffen besonders geeignet. In einer bevorzugten Ausführungsform des erfindungsgemäßen Verfahrens findet die Umsetzung des Kohlendioxids mit Wasserstoff bei einem Druck von 10 bis 50 bar und besonders bevorzugt zwischen 15 bis 30 bar statt. Vorzugsweise erfolgt die Umsetzung des Kohlendioxids mit Wasserstoff bei einer Temperatur von 300°C und einem Druck von 15 bar oder bei einer Temperatur von 350°C und einem Druck von 10 bar.

In einer bevorzugten Ausführungsform des erfindungsgemäßen Verfahrens ist das molare Verhältnis des eingesetzten Wasserstoffs zum Kohlendioxid 4 bis 8 und vorzugsweise 6.

Das im erfindungsgemäßen Verfahren verwendete Kohlendioxid kann durch Rauchgasabscheidung aus dem Abgas von Kraftwerken gewonnen werden, die elektrische Energie vorzugsweise aus fossilen Brennstoffen erzeugen. Das im erfindungsgemäßen Verfahren verwendete Wasserstoffgas kann durch Elektrolyseverfahren gewonnen werden, wobei diese bevorzugt mit Strom aus Photovoltaik-, Windkraftanlagen oder anderen CO₂-neutralen Anlagen betrieben werden.

Das erfindungsgemäße Verfahren zur Herstellung von Kohlenwasserstoffen aus Kohlendioxid und Wasserstoff kann in einem Festbett-, Wirbelschicht- oder Blasensäulenreaktor durchgeführt werden. Beispiele geeigneter Festbettreaktoren sind gasdurchströmte katalytische Festbettreaktoren, Blasensäulenreaktoren und Rohrbündelreaktoren. Im erfindungsgemäßen Verfahren wird ein gasdurchströmter katalytischer Festbettreaktor bevorzugt verwendet.

Bei der Umsetzung von Kohlendioxid und Wasserstoff im erfindungsgemäßen Verfahren wird eine Produktmischung erhalten, die Kohlenwasserstoffe, nicht-umgesetzten Wasserstoff, Wasser, nicht-umgesetztes Kohlendioxid, und Kohlenmonoxid enthalten kann. Das Kohlenmonoxid wird intermediär aus Kohlendioxid und Wasserstoff im RWGS gebildet. Bevorzugt enthält die Produktmischung C₅-C₁₅ und C₂-C₄ Kohlenwasserstoffe. Enthalten die C₂-C₄ Kohlenwasserstoffe C₂-C₄ Alkane, so können diese nach ihrer Abtrennung zu C₂-C₄ Alkenen, insbesondere Ethylen, Propylen und Butenen dehydriert werden.

C₅-C₁₅ Kohlenwasserstoffe umfassen lineare, verzweigte und zyklische Alkane und Alkene mit einer Kettenlänge von 5 bis 15 Kohlenstoffatomen. Beispiele der C₅-C₁₅ Alkane sind lineare, verzweigte und zyklische Isomere von Pentanen, Hexanen, Heptanen, Octanen, Nonanen, Decanen, Undecanen, Dodecanen, Tridecanen, Tetradecanen und Pentadecanen. Beispiele der C₅-C₁₅ Alkene umfassen lineare, verzweigte und zyklische Isomere von Pentenen, Hexenen, Heptenen, Octenen, Nonenen, Decenen, Undecenen, Dodecenen, Tridecenen, Tetradecenen und Pentadecenen. Bevorzugt sind die C₅-C₁₅ Kohlenwasserstoffe lineare und/oder verzweigte Alkane und Alkene.

C₂-C₄ Kohlenwasserstoffe umfassen C₂-C₄ Alkane und C₂-C₄ Alkene, die 2 bis 4 Kohlenstoffatome enthalten, wobei die C₄ Alkane und C₄ Alkene sowohl linear als auch verzweigt sein können. Beispiele der C₂-C₄ Kohlenwasserstoffe sind Ethan, Ethylen, Propan, Propylen, n-Butan, iso-Butan, 1-Buten, 2-Buten und 2-Methyl-1-propen. Butene umfassen n-Butan, iso-Butan, 1-Buten, 2-Buten und 2-Methyl-1-propen.

Mit dem erfindungsgemäßen Verfahren ist es möglich, aus CO₂ überwiegend die wertvollen C₂-C₁₅ Kohlenwasserstoffe herzustellen. Demnach sind im erfindungsgemäßen Verfahren die hergestellten Kohlenwasserstoffe vorzugsweise überwiegend C₂-C₁₅ Kohlenwasserstoffe, d.h. die hergestellten Kohlenwasserstoffe umfassen C₂-C₁₅ Kohlenwasserstoffe in einem Anteil von mindestens 50 mol%. Anders gesagt bestehen die Kohlenwasserstoffe der in dem erfindungsgemäßen Verfahren erhaltenen Produktmischung vorzugsweise zu ≥ 50 mol%, mehr bevorzugt zu > 70 mol% aus C₂-C₁₅ Kohlenwasserstoffen, bezogen auf die hergestellten Kohlenwasserstoffe.

Mit dem erfindungsgemäßen Verfahren ist es insbesondere möglich, einen größeren molaren Anteil von C₅-C₁₅ Kohlenwasserstoffen (die beispielsweise als Treibstoffe wie Benzin, Diesel und Kerosin verwendbar sind) als von C₂-C₄ Kohlenwasserstoffen (die ggf. nach Dehydrierung als Ausgangsstoffe für die chemische Industrie dienen) zu erhalten. Mit anderen Worten ist das molare Verhältnis von C₅-C₁₅ Kohlenwasserstoffen zu C₂-C₄ Kohlenwasserstoffen in der Produktmischung vorzugsweise > 1.

Als Nebenprodukte werden in dem erfindungsgemäßen Verfahren Methan und höhere Kohlenwasserstoffe (mit mehr als 16 Kohlenstoffatomen), insbesondere Wachse erhalten. Diese Nebenprodukte können in üblicher Weise genutzt werden. Beispielsweise kann das erzeugte Methan in dem erfindungsgemäßen Verfahren zu Heizzwecken dienen.

Das Produktgas wird vorzugsweise in einem Phasenabscheider auf 35 °C abgekühlt, um dabei Wasser in flüssiger Form abzuscheiden. Falls nötig kann überschüssiges Kohlendioxid in einem weiteren Schritt aus dem Produktgas durch Absorption entfernt werden. Dazu kann das Produktgas durch ein Medium, beispielsweise ein Lösungsmittel geleitet werden, das Kohlendioxid absorbiert. Alternativ kann das Kohlendioxid durch Druckwechsel-Adsorption (PSA-Adsorption) oder mittels einer Membran aus dem Produktgas entfernt werden. Das abgetrennte Kohlendioxidgas kann dem Eduktgasstrom, der Wasserstoffgas und Kohlendioxidgas umfasst, im erfindungsgemäßen Verfahren zugeführt werden.

Das Produktgas kann in einem Tieftemperaturtrennapparat oder mittels Tieftemperaturtrenntechnik in diejenigen Bestandteile aufgetrennt werden, die bei Raumtemperatur (20 °C) flüssig oder gasförmig sind, wobei ein Tieftemperaturwärmetauscher bevorzugt ist. Als Flüssigkeiten werden die C₅-C₁₅ Kohlenwasserstoffe erhalten, während die C₂-C₄ Kohlenwasserstoffe, Methan, überschüssiges Kohlenmonoxid und Wasserstoff gasförmig erhalten werden.

Optional kann überschüssiges Wasserstoffgas recycelt werden, indem Methan von dem Gasgemisch aus Methan, überschüssigem Kohlenmonoxid und Wasserstoff mittels Membran-Trenntechnologie oder Tieftemperaturabscheidung entfernt wird. Das recycelte Wasserstoffgas kann anschließend dem Eduktgasstrom des erfindungsgemäßen Verfahrens wieder zugeführt werden.

In einer bevorzugten Ausführungsform des erfindungsgemäßen Verfahrens wird der Katalysator vor der Verwendung in dem erfindungsgemäßen Verfahren mit Wasserstoff reduziert. Dazu wird der Katalysator beispielsweise bei 500°C in einem Inertgasstrom (z.B. Stickstoff), der Wasserstoff enthält, behandelt.

Der Katalysator, der im erfindungsgemäßen Verfahren verwendet wird, kann durch Fällung aus Metallsalzlösungen oder durch Vermahlen von Metalloxiden hergestellt werden. Auf diese Weise erhält man einen ungeträgerten Katalysator. Ein geträgerter Katalysator kann hergestellt werden, indem ein Katalysatorträger mit Metallsalzlösungen, z.B. wässrigen Metallnitratlösungen, imprägniert, beispielsweise tränkimprägniert, wird. Dabei wird vorzugsweise mit der Lösung begonnen, die die höchste Konzentration (z.B. in g/l) besitzt. Bevorzugt wird der imprägnierte Katalysatorträger nach jedem Imprägnierungsschritt getrocknet. Vorzugsweise wird den Katalysatoren während der Herstellung Oxalat zugesetzt, wobei das Oxalat bevorzugt in Form von Ammoniumoxalat oder Metalloxalat zugesetzt wird. Besonders bevorzugt ist Ammoniumoxalat. Wie sich zeigte, führt dies zu einer besseren Verteilung der Aktivkomponenten, insbesondere des Eisens, und der Promotoren im Katalysator bzw. auf dem Träger.

Die gefällten oder vermahlenen Metallsalze und die imprägnierten Katalysatorträger werden nach dem Trocknen vorzugsweise kalziniert. Das Kalzinieren kann beispielsweise in einem Muffelofen in stehender Luft (z.B. bei 350 °C) oder im Intertgasstrom (z. B. bei 400 °C) durchgeführt werden. Wenn ein Katalysator, der Oxalat enthält, kalziniert wird, findet eine vollständige Zersetzung (Verbrennung) des Oxalats statt, d.h. das Oxalat wird beim Kalzinieren rückstandslos entfernt.

Gemäß einem weiteren Aspekt betrifft die Erfindung den Katalysator als solchen. Der Begriff "Katalysator" verdeutlicht dabei, dass es sich um eine Zusammensetzung oder Verbindung handelt, die sich als Katalysator eignet, insbesondere in dem erfindungsgemäßen Verfahren zur Herstellung von Kohlenwasserstoffen.

Der erfindungsgemäße Katalysator umfasst als Katalysatorkomponenten, bezogen auf das Gesamtgewicht des Katalysators, mindestens 10 Gew.-%, vorzugsweise 10-50 Gew.-%, besonders bevorzugt 15 und 35 Gew.-% und ganz besonders bevorzugt 20-25 Gew.-% Eisen, sowie Kalium, Kupfer und mindestens eine weitere Komponente, ausgewählt aus Magnesium, Zink, Lanthan und Zirkonium. Zusätzlich kann der erfindungsgemäße Katalysator mindestens eines der folgenden Metalle umfassen: Mangan, Ruthenium, Aluminium und Cobalt.

Bevorzugte Ausführungsformen des erfindungsgemäßen Katalysators enthalten
- Eisen, Kalium, Kupfer und Lanthan;
- Eisen, Kalium, Kupfer und Zirkonium;
- Eisen, Kalium, Kupfer, Magnesium und Mangan;
- Eisen, Kalium, Kupfer, Zirkonium und Mangan;
- Eisen, Ruthenium, Kalium, Kupfer und Magnesium; oder
- Eisen, Ruthenium, Kalium, Kupfer und Zink.

Die Komponenten Eisen und Ruthenium stellen Aktivkomponenten dar.

Das Gewichtsverhältnis von Eisen zum Gesamtgewicht aller Metalle (Promotoren) aus der Gruppe Kalium, Kupfer, Magnesium, Zink, Lanthan, Aluminium, Zirkonium und Mangan im erfindungsgemäßen Katalysator ist vorzugsweise zwischen 1,5 und 15. Vorzugsweise liegt zumindest ein Teil der Metalle, besonders bevorzugt liegen sämtliche Metalle als Oxid vor.

Der erfindungsgemäße Katalysator kann sowohl geträgert als auch ungeträgert sein.

Falls der erfindungsgemäße Katalysator ein ungeträgerter ist, ist das Gesamtgewicht aller Metalle aus der Gruppe Kalium, Kupfer, Magnesium, Zink, Lanthan, Aluminium, Zirkonium und Mangan im Katalysator bezogen auf den Katalysator insgesamt vorzugsweise zwischen 15 und 50 Gew.-% und besonders bevorzugt zwischen 30 und 40 Gew.-%. Besonders bevorzugte Beispiele des ungeträgerten Katalysators der Erfindung sind:
11, 3La14, 1Cu70, 7Fe3, 9K; und
11, 6Mg11, 6Mn11, 6Cu58, 2Fe7, 0K,
wobei die Metalle als Oxid vorliegen können und die Anteile der Metalle in Gew.-%, bezogen auf das Gesamtgewicht aller Metalle in metallischer Form im Katalysator angegeben sind. Vorzugsweise liegt zumindest ein Teil der Metalle, besonders bevorzugt liegen sämtliche Metalle als Oxid vor.

Wenn der erfindungsgemäße Katalysator geträgert ist, dann kann der Träger aus der Gruppe bestehend aus Al₂O₃, Al₂O₃-MgO, TiO₂, La₂O₃, ZrO₂, ZrO₂-La₂O₃, ZrO₂-SiO₂, Zeolithen und Aluminium-Magnesiumhydroxycarbonaten ausgewählt sein. Al₂O₃-MgO bezeichnet Mischungen aus Al₂O₃ und MgO, wobei das Mischungsverhältnis beliebig sein kann. ZrO₂-La₂O₃ bezeichnet Mischungen aus ZrO₂ und La₂O₃, wobei das Mischungsverhältnis beliebig sein kann, bevorzugt liegt das Gewichtsverhältnis von La₂O₃ : ZrO₂ zwischen 5: 95 und 15: 85. ZrO₂-SiO₂ bezeichnet Mischungen aus ZrO₂ und SiO₂, wobei das Mischungsverhältnis beliebig sein kann. In den Aluminium-Magnesiumhydroxycarbonaten kann das Gewichtverhältnis zwischen Al₂O₃ und MgO beliebig sein. Bevorzugt liegt das Gewichtsverhältnis von Al₂O₃: MgO zwischen 30 : 70 (z.B. Pural MG70) und 50: 50. Die Aluminium-Magnesiumhydroxycarbonate umfassen amorphe und kristalline Formen, unter den kristallinen Formen ist Hydrotalcit bevorzugt.

Bevorzugte Katalysatorträger sind TiO₂, Al₂O₃ und Aluminium-Magnesiumhydroxycarbonat. Diese Trägermaterialien sind hinsichtlich der Ausbeute an C₅-C₁₅ Kohlenwasserstoffen vorteilhaft. Enthält der Katalysator TiO₂ als Träger, so wird eine hohe Ausbeute an C₅-C₁₅ Kohlenwasserstoffen in der Umsetzung von Kohlendioxid mit Wasserstoff in Gegenwart des Katalysators erzielt, während gleichzeitig wenig Methan gebildet wird.

Das Gesamtgewicht aller Metalle aus der Gruppe Kalium, Kupfer, Magnesium, Zink, Lanthan, Aluminium, Zirkonium und Mangan im geträgerten Katalysator beträgt vorzugsweise zwischen 1,5 und 20 Gew.-% und besonders bevorzugt zwischen 3 und 10 Gew.-% bezogen auf das Gewicht des Katalysators insgesamt.

Der geträgerte Katalysator der Erfindung ist vorzugsweise aus den folgenden Katalysatoren ausgewählt:
0,07Ru 1,27Mg 1,78Cu 20Fe 1,74K / Al₂O₃;
0,08Ru 0,91Mg 2,39Cu 19,23Fe 1,46K / Al₂O₃;
0,16Ru 1,11Mg 2,66Cu 19,26Fe 1,75K / Al₂O₃;
0,16Ru 1,11Mg 2,11Cu 19,23Fe 1,46K / Al₂O₃;
20Fe 2,66Al 2,14Cu 1,78K / TiO₂;
3,18La 4Cu 20Fe 1,1K / TiO₂;
0,63La 4Cu 20Fe 1,31K / Al₂O₃;
2,23Zr 4Cu 20Fe 1,73K / TiO₂;
0,1Ru 0,2Cu 0,29Zn 20Fe 1,4K / TiO₂;
3,27Zr 2,96Mn 1Cu 20Fe 1,48K / TiO₂;
20Fe 1,78Cu 1,74K 1,27Mg 0,07Ru / Aluminium-Magnesiumhydroxycarbonat; und
20Fe 4Mg 4Mn 4Cu 2,4K / Aluminium-Magnesiumhydroxycarbonat, wobei die Metalle als Oxid vorliegen können und die Anteile der Metalle in Gew.-%, bezogen auf die Summe des Gesamtgewichts aller Metalle in metallischer Form im Katalysator und des Trägers angegeben sind. Vorzugsweise liegt zumindest ein Teil der Metalle, besonders bevorzugt liegen sämtliche Metalle als Oxid vor.

Ein weiterer Aspekt der Erfindung betrifft die Verwendung der Katalysatoren, wie oben beschrieben, in einem der erfindungsgemäßen Verfahren (i) bis (iv).

Wie üblich, bezeichnet in den genannten Katalysatoren der Teil nach dem Symbol "/" den Träger. Alle Bestandteile vor "/" stellen die Aktivkomponenten und Promotoren dar, wobei diese zumindest teilweise in oxidischer Form vorliegen können. Beispielsweise bedeutet 0,07Ru 0,3Zn 20Fe 1,1K /TiO₂, dass der Katalysator TiO₂ als Träger, sowie 0,07 Gew.-% Ru, 0,3 Gew.-% Zn, 20 Gew.-% Fe und 1,1 Gew.-% K, alle bezogen auf die Summe des Gesamtgewichts aller Metalle in metallischer Form im Katalysator und des Katalysatorträgers, enthält.

Im Folgenden werden Beispiele gegeben, welche die Erfindung näher erläutern, jedoch nicht beschränken sollen.

### Beispiele

### Katalysator 1: 11,3La 14,1Cu 70,7Fe 3,9K

0,71 g La(NO₃)₃·6H₂O, 1,07 g Cu(NO₃)₂·3H₂O, 9,49 g Fe(NO₃)₃˙9H₂O und 0,20 g KNO₃ wurden in 100 ml dest. Wasser gelöst und auf 90 °C erwärmt. Zu der warmen Lösung wurde so lange eine 25%-ige NH₃-Lösung getropft bis keine weitere Fällung mehr zu beobachten war. Anschließend wurde die Mischung langsam bis zur Trockne eingedampft, im Trockenschrank bei 130 °C getrocknet und im Muffelofen bei 350 °C an Luft für 5 Stunden kalziniert.

### Katalysator 2: 11,6Mg 11,6Mn 11,6Cu 58,2Fe 7,0K

Katalysator 2 wurde analog zu der Herstellung von Katalysator 1 aus 2,45 g Mg(NO₃)₂˙6H₂O, 1,06 g Mn(NO₃)2˙4H2O, 0,88 g Cu(NO₃)₂·3H₂O, 7,81 g Fe(NO₃)₃˙9H₂O und 0,36 g KNO₃ hergestellt.

### Katalysator 3: 19,3Fe 1,5K 0,2Ru / TiO₂

Schritt 1: Auf 1g TiO₂ (Degussa Aerolyst 7708, Partikelgröße 0,1-0,25mm) wurden unter Schütteln bei Raumtemperatur 5ml einer wässrigen Fe(NO₃)₃-Lösung (Gehalt 48,7 g/l Fe) pipettiert, die Mischung auf 100 °C erwärmt und unter Schütteln bei 100 °C eingedampft und getrocknet.
Schritt 2: Auf die getrocknete Mischung von Schritt 1 wurden unter Schütteln bei Raumtemperatur 5ml einer wässrigen KNO₃-Lösung (Gehalt 3,69 g/l K) pipettiert, die Mischung auf 100 °C erwärmt und unter Schütteln bei 100 °C eingedampft und getrocknet.
Schritt 3: Auf die getrocknete Mischung von Schritt 2 wurden unter Schütteln bei Raumtemperatur 5ml einer wässrigen Ru(NO)(NO₃)₃-Lösung (Gehalt 0,49 g/l Ru) pipettiert, die Mischung auf 100 °C erwärmt und unter Schütteln bei 100 °C eingedampft und getrocknet.
Schritt 4: Die getrocknete Mischung wurde für 5 Stunden im Muffelofen bei 350 °C in Luft kalziniert.

### Katalysator 4: 19,2 Fe 1,9K 0,2Ru / TiO₂

Katalysator 4 wurde analog zur Herstellung von Katalysator 3 hergestellt, wobei in Schritt 2 der Gehalt der wässrigen KNO₃-Lösung 4,85 g/l K war.

### Katalysator 5: 0,1Ru 0,2Cu 0,3Zn 19,2Fe 1,3K / TiO₂

Katalysator 5 wurde analog zur Herstellung von Katalysator 3 hergestellt, wobei in Schritt 1 der Gehalt der wässrigen Fe(NO₃)₃-Lösung 48,8 g/l Fe war, in Schritt 2 der Gehalt der wässrigen KNO₃-Lösung 3,41 g/l K war, in Schritt 3 der Gehalt der wässrigen Zn(NO₃)₂-Lösung 0,71 g/l Zn war und Schritt 4 durch die folgenden Schritte 4 bis 6 ersetzt wurde:
Schritt 4: Auf die getrocknete Mischung von Schritt 3 wurden unter Schütteln bei Raumtemperatur 5ml einer wässrigen Cu(NO₃)₂-Lösung (Gehalt 0,48 g/l Cu) pipettiert, die Mischung auf 100 °C erwärmt und unter Schütteln bei 100 °C eingedampft und getrocknet.
Schritt 5: Auf die getrocknete Mischung von Schritt 4 wurden unter Schütteln bei Raumtemperatur 5ml einer wässrigen Ru(NO)(NO₃)₃-Lösung (Gehalt 0,26 g/l Ru) pipettiert, die Mischung auf 100 °C erwärmt und unter Schütteln bei 100 °C eingedampft und getrocknet.
Schritt 6: Die getrocknete Mischung wurde für 5 Stunden im Muffelofen bei 350 °C in Luft kalziniert.

### Katalysator 6: 0,07Ru 0,3Zn 19,2Fe 2,0K / TiO₂

Katalysator 6 wurde analog zur Herstellung von Katalysator 3 hergestellt, wobei in Schritt 1 der Gehalt der wässrigen Fe(NO₃)₃-Lösung 49,0 g/l Fe war, in Schritt 2 der Gehalt der wässrigen KNO₃-Lösung 5,13 g/l K war, in Schritt 3 der Gehalt der wässrigen Zn(NO₃)₂-Lösung 0,73 g/l Zn war und Schritt 4 durch die folgenden Schritte 4 und 5 ersetzt wurde:
Schritt 4: Auf die getrocknete Mischung von Schritt 3 wurden unter Schütteln bei Raumtemperatur 5ml einer wässrigen Ru(NO)(NO₃)₃-Lösung (Gehalt 0,18 g/l Ru) pipettiert, die Mischung auf 100 °C erwärmt und unter Schütteln bei 100 °C eingedampft und getrocknet.
Schritt 5: Die getrocknete Mischung wurde für 5 Stunden im Muffelofen bei 350 °C in Luft kalziniert.

### Katalysator 7: 2,2Zr 1,7Mn 2,4Zn 18,8Fe 1,6K / TiO₂

Katalysator 7 wurde analog zur Herstellung von Katalysator 5 hergestellt, wobei in Schritt 1 der Gehalt der wässrigen Fe(NO₃)₃-Lösung 51,3 g/l Fe war, in Schritt 2 der Gehalt der wässrigen KNO₃-Lösung 4,37 g/l K war, in Schritt 3 der Gehalt der wässrigen Zn(NO₃)₂-Lösung 6,68 g/l Zn war, in Schritt 4 der Gehalt der wässrigen ZrO(NO₃)₂-Lösung 5,91 g/l Zr war und in Schritt 5 der Gehalt der wässrigen Mn(NO₃)₂-Lösung 4,62 g/l Mn war.

### Katalysator 8: 0,97Cu 19,16Fe 1,63K / TiO₂

Katalysator 8 wurde analog zur Herstellung von Katalysator 3 hergestellt, wobei in Schritt 1 5ml einer wässrigen Lösung aus Fe(NO₃)₃ (Gehalt 49,1 g/l Fe) und 2 ml einer Ammoniumoxalatlösung (Gehalt 25,15 g/l Oxalat) verwendet wurden, in Schritt 2 der Gehalt der wässrigen KNO₃-Lösung 4,17 g/l K war und in Schritt 3 der Gehalt der wässrigen Cu(NO₃)₂-Lösung 2,48 g/l Cu war.

### Katalysator 9: 2,1Zr 3,8Cu 18,8Fe 1,6K / TiO₂

Katalysator 9 wurde analog zur Herstellung von Katalysator 6 hergestellt, wobei in Schritt 1 der Gehalt der wässrigen Fe(NO₃)₃-Lösung 51,2 g/l Fe war, in Schritt 2 der Gehalt der wässrigen KNO₃-Lösung 4,42 g/l K war, in Schritt 3 der Gehalt der wässrigen Cu(NO₃)₂-Lösung 10,24 g/l Cu war und in Schritt 4 der Gehalt der wässrigen ZrO(NO₃)₂-Lösung 5,71 g/l Zr war.

### Katalysator 10 : 3,1Zr 2,8Mn 0,9Cu 18,8Fe 1,4K / TiO₂

Katalysator 10 wurde analog zur Herstellung von Katalysator 5 hergestellt, wobei in Schritt 1 der Gehalt der wässrigen Fe(NO₃)₃-Lösung 51,5 g/l Fe war, in Schritt 2 der Gehalt der wässrigen KNO₃-Lösung 3,80 g/l K war, in Schritt 3 der Gehalt der wässrigen ZrO(NO₃)₂-Lösung 8,43 g/l Zr war, in Schritt 4 der Gehalt der wässrigen Mn(NO₃)₂-Lösung 7,62 g/l Mn war und in Schritt 5 der Gehalt der wässrigen Cu(NO₃)₂-Lösung 2,57 g/l Cu war.

### Ergebnisse der katalytischen Tests der Katalysatoren 1-10

Die Katalysatoren 1-10 wurden für die CO₂-Hydrierung zu Kohlenwasserstoffen getestet. Dazu wurden jeweils 0,3 g eines Katalysators in einen Edelstahlreaktor (5 mm Innendurchmesser) eingebaut. Der Katalysator wurde vor Versuchsbeginn in einem 10% H₂/N₂-Strom für 2 h bei 500 °C und 10 bar reduziert und anschließend in diesem Gas auf Reaktionstemperatur abgekühlt. Der katalytische Test erfolgte unter den in Tabelle 1 genannten Reaktionsbedingungen. Die Produktmischung wurde gaschromatographisch analysiert, und die Umsätze (X) und Ausbeuten (Y) eduktbasiert und die Selektivität (S) produktbasiert berechnet.

Die Prüfung der katalytischen Eigenschaften der Katalysatoren 1 - 10 in Bezug auf die Hydrierung von Kohlendioxid mit Wasserstoff erfolgte in einem Festbettreaktor (Edelstahlrohr, Innendurchmesser 5 mm). Alle Katalysatoren wurden in einer Kornfraktion von 100-250 µm eingesetzt, um Stofftransporteinflüsse zu minimieren und eine Pfropfenströmung ("plug-flow"-Strömung) im Reaktor zu gewährleisten.

Die Analyse des Produktgases erfolgte durch einen on-line gekoppelten Gaschromatographen in der Gasphase. Dazu wurde ein Betriebsdruck von 10 bar gewählt und zum anderen wurden alle Rohrleitungen und Ventile nach dem Reaktor sowie das GC-Einlasssystem auf mindestens 150°C beheizt. Zusätzlich wurden in die Abgasleitung ein Heißgasabscheider (150°C) und ein Kaltgasabscheider (Raumtemperatur) jeweils mit integriertem Aerosolfilter integriert. Es wurde die zweidimensionale Kapillar-Gaschromatographie angewandt.

Folgende Komponenten wurden analysiert:
● Mittels Wärmeleitfähigkeitsdetektor (WLD): CO₂, Ethylen, Ethan, Wasser, H₂, N₂, Methan, CO
● Mittels Flammenionisationsdetektor (FID): Methan, C₂-C₄ (alle Alkan- und Olefin-Isomere), C₅-C₁₈ (n-Alkane und n-1-Olefine), die anderen C₅-C₁₈-Isomere wurden analysiert, aber nicht strukturell zugeordnet.

Für die Analyse wurde ein Agilent 7890 Gaschromatograph mit zwei unabhängigen Kanälen benutzt. Beide Kanäle wurden mit Helium als Trägergas betrieben. Ein Kanal des Gaschromatographen war mit einer PorapakQ-Säule und einer PoraPlotQ-Säule ausgestattet. Die Säulen wurden mit einem Temperaturprogramm von der Starttemperatur 50°C auf 200°C aufheizt. Der zweite Kanal war mit einer FFAP-Säule und einer Al₂O₃/KCl-Säule (25m x 0.32mm, 5µm Filmdicke) ausgestattet, wobei die FFAP-Säule bei einer Temperatur von 50-200°C betrieben wurde. Die Al₂O₃/KCl-Säule wurde mit einem Temperaturprogram von 80°C bis 200°C betrieben.

**Tabelle 1: Ergebnisse der katalytischen Tests. Reaktionsbedingungen: p=15 bar, H₂:CO₂=6, GHSV=1320 ml g_{Kat}⁻¹ h⁻¹**

| Katalysator | T in °C | X ( CO₂) | X(H₂) | Y(C₅-C₁₅) | Y(C₂-C₄) | Y(CO) | Y(CH₄) | S(C₅-C₁₅) | Olefinanteil (C₂-C₄) |
|---|---|---|---|---|---|---|---|---|---|
| 1 | 300 | 62% | 33% | 23% | 23% | 2% | 8% | 41% | 86% |
| 2 | 300 | 62% | 32% | 22% | 22% | 2% | 8% | 40% | 86% |
| 3 | 350 | 66% | 31% | 18% | 20% | 5% | 12% | 32% | 82% |
| 4 | 350 | 65% | 31% | 18% | 19% | 5% | 11% | 34% | 83% |
| 5 | 350 | 65% | 33% | 17% | 19% | 5% | 11% | 33% | 82% |
| 6 | 350 | 64% | 32% | 18% | 19% | 5% | 10% | 35% | 85% |
| 7 | 350 | 63% | 30% | 17% | 20% | 5% | 10% | 32% | 80% |
| 8 | 350 | 63% | 31% | 17% | 19% | 5% | 11% | 33% | 83% |
| 9 | 350 | 69% | 35% | 17% | 21% | 4% | 14% | 30% | 79% |
| 10 | 300 | 46% | 23% | 16% | 15% | 5% | 5% | 40% | 80% |

| | | | | | | | | | |
|---|---|---|---|---|---|---|---|---|---|
| Olefinanteil = Anteil an C₂-C₄ Olefinen in Mol-% bezogen auf alle C₂-C₄ Kohlenwasserstoffe (Y(C₂-C₄)) | | | | | | | | | |

### Katalysatoren 11 - 67

Im Syntheseroboter Sophas (Zinsser Analytics) wurden die Katalysatoren 11-67 durch Imprägnierung von Trägermaterialien hergestellt. Falls mehrere Komponenten auf einem Träger enthalten sind, wurde die Imprägnierung sequentiell, beginnend mit der Komponente mit der höchsten Konzentration, durchgeführt, wobei nach jedem Imprägnierschritt bei einer Temperatur von 100°C getrocknet wurde. Dadurch wurde sichergestellt, dass alle Katalysatorbestandteile auf dem Trägermaterial abgeschieden wurden.

Nach abschließender Trocknung im Syntheseroboter wurden die Katalysatoren im Muffelofen für 5 h in stehender Luft bei 350°C kalziniert (Heizrate 5 K/min). Die oxalathaltigen Proben wurden abweichend davon wegen der sonst zu schnellen Oxalatzersetzung mit nur 2,5 K/min aufgeheizt.

Ausgangsmaterialien:
- Trägermaterialien: γ-Al₂O₃ (Condea), TiO₂ (Degussa Aerolyst 7708), SiO₂ (Degussa Aerolyst 355), Aluminium-Magnesiumhydroxycarbonat mit einem Gewichtsverhältnis Al₂O₃ : MgO von 30 : 70 (Pural MG70)

In Tabelle 2 sind die Zusammensetzungen der Katalysatoren 11 - 67 zusammengefasst. Der Anteil des jeweiligen Metalls in metallischer Form ist in Gew.-% angegeben und bezieht sich auf das Gesamtgewicht aller Metalle in metallischer Form im Katalysator und des Katalysatorträgers.

**Tabelle 2: Zusammensetzungen der Katalysatoren 11 - 67**

| Katalysator | Träger | Fe in % | Co in % | Ru in % | Al in % | La in % | Mg in % | Zr in % | Mn in % | Cu in % | Zn in % | K in % | Oxalat * |
|---|---|---|---|---|---|---|---|---|---|---|---|---|---|
| 11 | TiO₂ | 20 | | | | 3,2 | | | | 4,0 | | 1,1 | nein |
| 12 | TiO₂ | 20 | | 0,1 | | | | | | | 0,3 | 2,1 | nein |
| 13 | Al₂O₃ | 20 | | | | 0,6 | | | | 4,0 | | 1,3 | nein |
| 14 | Al₂O₃ | 18 | 2 | | | | 3,4 | | | 2,9 | | 1,6 | ja |
| 15 | Al₂O₃ | 17 | 3 | | | | 3,4 | | | 1,9 | | 1,6 | ja |
| 16 | TiO₂ | 20 | | | | 2,8 | | | | 4,0 | | 1,1 | nein |
| 17 | Al₂O₃ | 20 | | | | | | 2,8 | 1,9 | 3,0 | | 1,7 | ja |
| 18 | Al₂O₃ | 20 | | 0,1 | | | | | 0,1 | | | 1,5 | nein |
| 19 | Al₂O₃ | 18 | 2 | 0,2 | | | | | | | | 2,2 | nein |
| 20 | Al₂O₃ | 10 | | 0,1 | | | | | | 1,1 | 1,7 | 0,9 | ja |
| 21 | Al₂O₃ | 20 | | | | | | 2,0 | | 2,3 | | 1,7 | nein |
| 22 | TiO₂ | 10 | | | | | | | 1,3 | 1,7 | 1,1 | 0,7 | ja |
| 23 | Ti02 | 10 | | | | | | | 2,0 | 2,0 | 2,0 | 0,6 | ja |
| 24 | Al₂O₃ | 10 | | 0,1 | | | | | | | | 1,2 | nein |
| 25 | Al₂O₃ | 10 | | 0,1 | | | | | | 2,0 | 1,9 | 0,8 | ja |
| 26 | Al₂O₃ | 10 | | | | | 1,0 | | | 2,0 | | 0,8 | ja |
| 27 | TiO₂ | 20 | | | | | | 2,2 | | 4,0 | | 1,7 | nein |
| 28 | Al₂O₃ | 20 | | | | 1,9 | | | | 3,7 | | 1,5 | ja |
| 29 | Al₂O₃ | 10 | | 0,1 | | | | | | | 2,0 | 1,2 | nein |
| 30 | Ti02 | 20 | | | | 0,3 | | 2,5 | 2,5 | | 0,9 | 1,7 | nein |
| 31 | TiO₂ | 20 | | | | | | | | 1,0 | | 1,7 | ja |
| 32 | TiO₂ | 20 | | | | | | 2,3 | 0,8 | 3,6 | | 1,1 | ja |
| 33 | TiO₂ | 20 | | | | | | 3,3 | 3,0 | 1,0 | | 1,5 | nein |
| 34 | TiO₂ | 10 | | 0,1 | | | | 1,0 | | 2,0 | | 1,2 | nein |
| 35 | TiO₂ | 20 | | 0,1 | | | | | | 2,1 | 0,1 | 1,0 | ja |
| 36 | TiO₂ | 20 | | | | | | 3,9 | | 1,0 | | 2,4 | nein |
| 37 | TiO₂ | 20 | | | | | | 2,3 | 1,8 | | 2,6 | 1,7 | nein |
| 38 | TiO₂ | 10 | | 0,1 | | | | | | | 2,0 | 1,2 | nein |
| 39 | Al₂O₃ | 10 | | | | | 2,0 | | 2, 0 | 2,0 | | 1,2 | nein |
| 40 | TiO₂ | 20 | | 0,1 | | | | | | | 0,3 | 1,1 | nein |
| 41 | TiO₂ | 10 | | 0,1 | | | | 0,6 | | 2,0 | | 0,8 | nein |
| 42 | TiO₂ | 20 | | | | | | | | | 3,0 | 1,7 | ja |
| 43 | TiO₂ | 20 | | 0,07 | | | | | | | 0,3 | 2,1 | nein |
| 44 | TiO₂ | 20 | | | | | | 2,23 | | 4 | | 1,73 | nein |
| 45 | TiO₂ | 20 | | 0,07 | | | | | | | 0,3 | 1,1 | nein |
| 46 | TiO₂ | 20 | | | | 0,32 | | 2,51 | | 0,88 | | 1,7 | nein |
| 47 | TiO₂ | 19,16 | | | | | | | | 0,97 | | 1,63 | nein |
| 48 | Al₂O₃ | 20 | | 0,07 | | | 1,27 | | | 1,78 | | 1,74 | nein |
| 49 | Al₂O₃ | 20 | | 0,1 | | | 0,26 | | | | | 2,1 | nein |
| 50 | Al₂O₃ | 19,23 | | 0,08 | | | 0,91 | | | 2,39 | | 1,46 | nein |
| 51 | Al₂O₃ | 19,26 | | 0,16 | | | 1,11 | | | 2,66 | | 1,75 | nein |
| 52 | Al₂O₃ | 19,23 | | 0,16 | | | 1,11 | | | 2,11 | | 1,46 | nein |
| 53 | TiO₂ | 20 | | | 2,66 | | | | | 2,14 | | 1,78 | nein |
| 54 | TiO₂ | 20 | | | | 3,18 | | | | 4 | | 1,1 | nein |
| 55 | Al₂O₃ | 18,08 | 1,92 | 0,2 | | | | | | | | 2,17 | nein |
| 56 | Al₂O₃ | 20 | | 0,02 | | | 1,02 | 0,1 | | | | 0,83 | nein |
| 57 | Al₂O₃ | 20 | | | | 0,63 | | | | 4 | | 1,31 | nein |
| 58 | TiO₂ | 20 | | 0,2 | | | | | | | | 1,99 | nein |
| 59 | TiO₂ | 20 | | 0,2 | | | | | | | | 1,52 | nein |
| 60 | TiO₂ | 20 | | 0,1 | | | | | | 0,2 | 0,29 | 1,4 | nein |
| 61 | TiO₂ | 20 | | 0,2 | | | | | | | | 0,98 | nein |
| 62 | TiO₂ | 20 | | | | | | 3,27 | 2,96 | 1 | | 1,48 | nein |
| 63 | TiO₂ | 20 | | 0,2 | | | | | | | | 1,16 | nein |
| 64 | Ti02 | 20 | | | | | | 3,27 | 2,96 | 1 | | 1,48 | nein |
| 65 | Pural MG70 | 20 | | 0,07 | | | 1,27 | | | 1,78 | | 1,74 | nein |
| 66 | Pural MG70 | 20 | | | | | 4 | | 4 | 4 | | 2,4 | nein |
| 67 | Pural MG70 | 20 | | 0,07 | | | | | | | 0,3 | 2,1 | nein |

| | | | | | | | | | | | | | |
|---|---|---|---|---|---|---|---|---|---|---|---|---|---|
| *: Oxalat wurde in der Herstellung des Katalysators verwendet, in der Zusammensetzung des kalzinierten Katalysators ist Oxalat jedoch nicht enthalten. | | | | | | | | | | | | | |

### Ergebnisse der katalytischen Tests der Katalysatoren 11 - 67

Die Prüfung der katalytischen Eigenschaften der Katalysatoren 11 - 67 in Bezug auf die Hydrierung von Kohlendioxid mit Wasserstoff sowie die Analyse der Produktgase erfolgte analog zu der Prüfung und Analyse der Katalysatoren 1 - 10.

Die Ergebnisse der Umsetzung von Kohlendioxid mit Wasserstoff in Gegenwart der Katalysatoren 11 - 67 sind in den folgenden Tabellen 3 bis 7 aufgelistet. Die Umsätze (X) und Ausbeuten (Y) wurden eduktbasiert und die Selektivität (S) produktbasiert berechnet.

**Tabelle 3: Ergebnisse der katalytischen Tests. Reaktionsbedingungen: p=10 bar, T = 300°C, H₂:CO₂:N₂=71:24:5, GHSV=1320 ml g_{Kat}⁻¹ h⁻¹**

| Kat. | X(CO₂) | Y (C1) | Y(C₂-C₄) | Y(C₅-C₁₅) | Y(CO) | S(C₅-C₁₅) | C₅-C₁₅-Anteil an den KW | Olefin-anteil (C₂) | Olefin-anteil (C₃) | Olefin-anteil (C₄) |
|---|---|---|---|---|---|---|---|---|---|---|
| 11 | 27% | 1% | 5% | 9% | 7% | 40% | 59% | 44% | 82% | 79% |
| 12 | 27% | 2% | 5% | 8% | 7% | 37% | 55% | 47% | 82% | 79% |
| 13 | 32% | 6% | 11% | 7% | 4% | 26% | 30% | 1% | 15% | 34% |
| 14 | 30% | 5% | 11% | 7% | 5% | 25% | 30% | 8% | 55% | 60% |
| 15 | 30% | 6% | 11% | 7% | 5% | 24% | 29% | 7% | 52% | 59% |
| 16 | 27% | 1% | 5% | 7% | 5% | 35% | 54% | 55% | 82% | 81% |
| 17 | 30% | 5% | 10% | 7% | 5% | 25% | 30% | 4% | 31% | 47% |
| 18 | 30% | 5% | 10% | 7% | 5% | 25% | 30% | 38% | 71% | 76% |
| 19 | 31% | 6% | 11% | 7% | 5% | 24% | 29% | 37% | 79% | 77% |
| 20 | 29% | 6% | 12% | 7% | 4% | 23% | 27% | 2% | 22% | 40% |
| 21 | 30% | 6% | 11% | 6% | 5% | 23% | 27% | 2% | 30% | 47% |
| 22 | 23% | 2% | 4% | 6% | 9% | 30% | 51% | 14% | 65% | 62% |
| 23 | 24% | 2% | 4% | 6% | 9% | 29% | 51% | 10% | 57% | 57% |
| 24 | 27% | 5% | 10% | 6% | 5% | 24% | 30% | 33% | 73% | 75% |
| 25 | 30% | 6% | 12% | 6% | 4% | 22% | 26% | 2% | 13% | 29% |
| 26 | 29% | 6% | 11% | 6% | 4% | 23% | 27% | 6% | 42% | 54% |
| 27 | 25% | 1% | 4% | 6% | 9% | 30% | 55% | 72% | 84% | 83% |
| 28 | 26% | 4% | 8% | 6% | 6% | 24% | 32% | 2% | 24% | 43% |
| 29 | 28% | 5% | 10% | 6% | 5% | 23% | 28% | 26% | 66% | 71% |

**Tabelle 4: Ergebnisse der katalytischen Tests. Reaktionsbedingungen: p=10 bar, T = 300°C, H₂:CO₂:N₂=71:24:5, GHSV=1320 ml g_{Kat}⁻¹ h⁻¹**

| Kat. | X(CO₂) | S(C1) | S(C₂-C₄) | S(C₅-C₁₅) | S(CO) | Y(C₅-C₁₅)- Y(C1) |
|---|---|---|---|---|---|---|
| 30 | 24% | 6% | 19% | 26% | 49% | 4% |
| 31 | 23% | 6% | 17% | 25% | 52% | 3% |
| 32 | 26% | 8% | 24% | 25% | 42% | 3% |
| 33 | 25% | 9% | 23% | 24% | 43% | 3% |
| 34 | 23% | 8% | 20% | 23% | 49% | 3% |
| 35 | 25% | 10% | 26% | 24% | 40% | 3% |
| 36 | 21% | 5% | 15% | 19% | 61% | 3% |
| 37 | 22% | 7% | 19% | 21% | 53% | 3% |
| 38 | 22% | 7% | 20% | 21% | 52% | 2% |
| 39 | 24% | 14% | 33% | 24% | 29% | 2% |
| 40 | 22% | 9% | 21% | 20% | 50% | 2% |
| 41 | 23% | 12% | 26% | 22% | 40% | 2% |
| 42 | 18% | 6% | 15% | 17% | 62% | 2% |

**Tabelle 5: Ergebnisse der katalytischen Tests. Reaktionsbedingungen: p=10 bar, T = 350°C, H₂:CO₂=3, GHSV=660 ml g_{Kat}⁻¹ h⁻¹**

| Kat. | Y(C₅-C₁₅) -Y(C1) | X(CO₂) | X(^{H}2) | Y(CO) | Y(CH4) | Y(C₂-C₄) | Y(C₅-C₁₅) | S(C₂-C₄) | S(C₅-C₁₅) |
|---|---|---|---|---|---|---|---|---|---|
| 43 | 5,8% | 42% | 41% | 6,0% | 5,5% | 9,2% | 11,3% | 29% | 35% |
| 44 | 5,8% | 42% | 40% | 6,0% | 5,1% | 9,4% | 10,6% | 30% | 34% |
| 45 | 5,5% | 41% | 41% | 5,4% | 5,6% | 10,5% | 11,2% | 32% | 34% |
| 46 | 4,8% | 39% | 38% | 6,3% | 4,9% | 9,6% | 9,7% | 31% | 32% |
| 47 | 4,9% | 38% | 36% | 7,1% | 4,5% | 8,5% | 9,4% | 29% | 32% |

**Tabelle 6: Ergebnisse der katalytischen Tests. Reaktionsbedingungen: p=15 bar, T = 350°C, H₂:CO₂=6, GHSV=1320 ml g_{Kat}⁻¹ h⁻¹**

| Kat. | Y(C₅-C₁₅) in % | | | |
|---|---|---|---|---|
| | 300°C, H₂:CO₂=3 | 300°C, H₂:CO₂=6 | 350°C, H₂:CO₂=3 | 350°C, H₂: CO₂=6 |
| 48 | 10,1 | 15,6 | 10,7 | 15,0 |
| 49 | 7,0 | 12,2 | 9,5 | 12,1 |
| 50 | 9,0 | 13,0 | 10,6 | 10,7 |
| 51 | 8,9 | 14,2 | 9,9 | 12,0 |
| 52 | 9,4 | 13,5 | 11,5 | 12,9 |
| 53 | 7,2 | 13,9 | 10,9 | 18,1 |
| 54 | 9,4 | 11,5 | 6,7 | 10,7 |
| 55 | 8,3 | 11,7 | 8,9 | 11,1 |
| 56 | 7,6 | 11,3 | 9,6 | 12,7 |
| 57 | 10,6 | 14,3 | 11,2 | 13,1 |

**Tabelle 7: Ergebnisse der katalytischen Tests. Reaktionsbedingungen: p=15 bar, H₂:CO₂=6, GHSV=1320 ml g_{Kat}⁻¹ h⁻¹**

| Kat. | T in °C | X(CO₂) | X(H₂) | Y(C₅-C₁₅) | Y(C₂-C₄) | Y(CO) | Y(CH₄) | S(C₅-C₁₅) | Olefinanteil (C₂-C₄) |
|---|---|---|---|---|---|---|---|---|---|
| 58 | 350 | 65% | 31% | 18% | 19% | 5% | 11% | 34% | |
| 59 | 350 | 66% | 31% | 18% | 20% | 5% | 12% | 32% | |
| 60 | 350 | 65% | 33% | 17% | 19% | 5% | 11% | 33% | |
| 61 | 300 | 50% | 26% | 17% | 17% | 4% | 6% | 38% | |
| 62 | 300 | 46% | 23% | 16% | 15% | 5% | 5% | 40% | |
| 63 | 350 | 67% | 34% | 16% | 21% | 4% | 13% | 29% | |
| 64 | 350 | 64% | 31% | 16% | 21% | 5% | 12% | 30% | |
| 65 | 350 | 72% | 38% | 22,4% | 24,2% | 3,4% | 17,2% | 33,2% | 83% |
| 66 | 350 | 70% | 36% | 23,1% | 23,0% | 3,8% | 13,8% | 36,1% | 84% |
| 67 | 350 | 73% | 38% | 22,6% | 23,7% | 3,3% | 17,3% | 33,6% | 85% |

## Patentansprüche

1. Verfahren zur Herstellung von Kohlenwasserstoffen aus Kohlendioxid und Wasserstoff, bei dem Kohlendioxid mit Wasserstoff in Gegenwart eines Katalysators umgesetzt wird, wobei der Katalysator Eisen und mindestens ein Alkalimetall enthält und ferner mindestens eines der folgenden Merkmale (i) bis (iii) erfüllt:
(i) der Katalysator enthält, bezogen auf das Gesamtgewicht des Katalysators, Eisen in einer Menge von mindestens 10 Gew.-%, vorzugsweise von 15 und 35 Gew.-%, und ferner Kupfer und mindestens einen weiteren Vertreter, ausgewählt aus Magnesium, Zink, Lanthan und Zirkonium;
(ii) der Katalysator umfasst TiO₂ und/oder ein Aluminium-Magnesiumhydroxycarbonat als Katalysatorträger;
(iii) der Katalysator enthält ferner Ruthenium und/oder Cobalt.

2. Verfahren gemäß Anspruch 1, bei dem die Umsetzung des Kohlendioxids mit Wasserstoff in Gegenwart des Katalysators bei einer Temperatur von 150 bis 400°C, vorzugsweise 320 bis 370°C, und einem Druck von 10 bis 50 bar, vorzugsweise 15 bis 30 bar, erfolgt.

3. Verfahren gemäß Anspruch 1 oder 2, bei dem das molare Verhältnis des eingesetzten Wasserstoffs zum Kohlendioxid 4 bis 8 ist.

4. Verfahren gemäß mindestens einem der Ansprüche 1 bis 3, bei dem eine Produktmischung erhalten wird, die Kohlenwasserstoffe, Kohlenmonoxid, nicht-umgesetztes Kohlendioxid und nicht-umgesetzten Wasserstoff enthält, und nach Abtrennung der Kohlenwasserstoffe nicht-umgesetztes Kohlendioxid, nicht-umgesetzter Wasserstoff und Kohlenmonoxid wieder der Eduktmischung zugeführt werden.

5. Verfahren gemäß mindestens einem der Ansprüche 1 bis 4, bei dem die Kohlenwasserstoffe C₂-C₄ Kohlenwasserstoffe umfassen.

6. Verfahren gemäß Anspruch 5, bei dem die C₂-C₄ Kohlenwasserstoffe C₂-C₄ Alkane und C₂-C₄ Alkene enthalten und die C₂-C₄ Alkane nach ihrer Abtrennung anschließend zu C₂-C₄ Alkenen, insbesondere zu Ethylen, Propylen und Butenen, dehydriert werden.

7. Verfahren gemäß mindestens einem der Ansprüche 1 bis 4, bei dem die Kohlenwasserstoffe C₅-C₁₅ Kohlenwasserstoffe umfassen.

8. Verfahren gemäß mindestens einem der Ansprüche 1 bis 7, bei dem das mindestens eine Alkalimetall in dem Katalysator Kalium ist.

9. Verfahren gemäß mindestens einem der Ansprüche 1 bis 8, bei dem der Katalysator enthält:
● Magnesium;
● Zink;
● Zirkonium, Mangan und Zink;
● Kupfer;
● Kupfer und einen weiteren Vertreter, ausgewählt aus der Gruppe, bestehend aus Aluminium, Lanthan, Zirkonium, Zink und Magnesium;
● Kupfer, Magnesium und Mangan; oder
● Kupfer, Zirkonium und Mangan.

10. Verfahren gemäß mindestens einem der Ansprüche 1 bis 9, bei dem der Katalysator das Merkmal (i) oder (iii) erfüllt und einen Katalysatorträger umfasst.

11. Verfahren gemäß Anspruch 10, bei dem der Katalysatorträger ausgewählt ist aus der Gruppe, bestehend aus Al₂O₃, Al₂O₃-MgO, TiO₂, La₂O₃, ZrO₂, ZrO₂-La₂O₃, ZrO₂-SiO₂, Zeolithen und Aluminium-Magnesiumhydroxycarbonaten.

12. Verfahren gemäß Patentanspruch 10, bei dem der Katalysatorträger ausgewählt ist aus der Gruppe, bestehend aus TiO₂, Al₂O₃ und Aluminium-Magnesiumhydroxycarbonat.

13. Katalysator der als Katalysatorkomponenten, bezogen auf das Gesamtgewicht des Katalysators, mindestens 10 Gew.-%, vorzugsweise 15-35 Gew.-% Eisen, sowie Kalium, Kupfer und mindestens eine weitere Komponente, ausgewählt aus Magnesium, Zink, Lanthan und Zirkonium umfasst.

14. Katalysator gemäß Anspruch 13, der einen Katalysatorträger umfasst, auf dem die Katalysatorkomponenten aufgebracht sind.

15. Katalysator gemäß Anspruch 14, wobei der Katalysatorträger ausgewählt ist aus der Gruppe, bestehend aus TiO₂, Al₂O₃ und/oder Aluminium-Magnesiumhydroxycarbonat.
